# EUROPEAN PATENT SPECIFICATION

(11) **EP 3 183 244 B1**
(45) Date of publication and mention of the grant of the patent: **10.04.2019**
(21) Application number: 15749841.1
(22) Date of filing: 14.08.2015
(51) Int. Cl.: C07D 401/04

(54) **PROCESS FOR MAKING CRYSTALLINE FORM A OF LENALIDOMIDE**
VERFAHREN ZUR HERSTELLUNG VON LENALIDOMIDFORM-A
PROCÉDÉ POUR FABRIQUER FORME A DE LÉNALIDOMIDE

(30) Priority: 19.08.2014 WO PCT/EP2014/067637
(43) Date of publication of application: 28.06.2017
(73) Proprietor: Synthon BV, 6545 CM Nijmegen (NL)
(72) Inventor: CASTULIK, Jakub, 67817 Blansko (CZ); VYKLICKY, Libor, 67817 Blansko (CZ); JELINEK, Zdenek, 67817 Blansko (CZ)
(74) Representative: Tejedor Vinent, Henar
(86) International application number: PCT/EP2015/068774
(87) International publication number: WO 2016/026785

(56) References cited:
- WO-A1-2011/027326
- WO-A1-2015/057043
- WO-A2-2010/100476
- IN-A1- 47C HE2 006

## Description

### BACKGROUND OF THE INVENTION

The present invention relates to an improved process for making the compound lenalidomide, especially in its specific crystalline Form A.

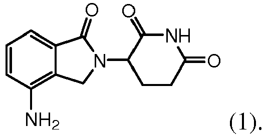

Lenalidomide of formula (1) is a pharmaceutically active compound that antagonizes tumor necrosis factor α and is thought to elevate levels of adenosine 3',5'-cyclic monophosphate. Lenalidomide and various structural analogues are useful in the treatment of a wide range of conditions including autoimmune disease and cancer.

The marketed pharmaceutical compositions comprising lenalidomide as the active substance are hard capsules. They are marketed under brand name Revlimid by Celgene.

Herein under, the name lenalidomide will be used as the generic name for racemic 3-(4-Amino-1-oxo -1, 3-dihydro-2H-isoindol- 2-yl)piperidine-2, 6-dione, unless indicated to the contrary.

Lenalidomide was first disclosed in WO98/03502 by Celgene Company.

WO2005/023192 describes various solid state forms of lenalidomide: crystalline forms A, B, C, D, E, F, G, H, WO2010/061209 discloses anhydrous crystalline lenalidomide, WO2011/034504 discloses, Form I, CN101817813 discloses Form IV, WO2011/111053 discloses Form I, CN102453021 discloses a Form 1, CN102453020 discloses Form II, WO2012/127493 discloses Form H1 and WO2009/114601 discloses amorphous lenalidomide.

Among the solid state forms of lenalidomide, the crystalline Form A of WO2005/023192 is preferred in pharmaceutical industry as it is a sufficiently stable, crystalline and non-hygroscopic with a good processability and compatibility with pharmaceutical excipients particularly, for making solid state dosage forms.

Processes for preparation of lenalidomide form A have been disclosed in several prior art documents.

WO2005/023192 discloses, without any details, a process for preparation of the form A by a crystallization of crude lenalidomide from various non-aqueous solvent including 1-butanol, butyl acetate, ethanol, ethyl acetate, methanol, methyl ethyl ketone and tetrahydrofurane.

Lenalidomide may be prepared, in general, by a reduction of the nitro-group in 3-(4-nitro-1-oxo-1,3-dihydro-2*H*-isoindol-2-yl)-2,6-piperidinedione of formula (2),

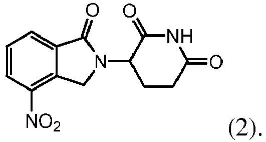

WO2011/027326 discloses a process comprising hydrogenation the compound of formula (2) in N,N-dimethylformamide and, optionally, methanol in the presence of Pd/C, by hydrogen at the pressure of 50-60 psi. The reaction mixture was filtered, and the solvent from the filtrate was distilled off. After stirring the residual solid in methanol or 2-propanol, lenalidomide was obtained. The drawback of this process is the necessity of a distillation step, the use of a second solvent for lenalidomide form A crystallization and the use of a high pressure of hydrogen for the hydrogenation step.

WO2011/033468 discloses a process of hydrogenation of 3-(4-nitro-1-oxo-1,3-dihydro-2*H*-isoindol-2-yl)-2,6-piperidinedione of formula (2) in N,N-dimethylformamide in the presence of Pd/C with hydrogen at the pressure of 50-60 psi. After the reaction, the N,N-dimethylformamide is distilled off and lenalidomide in crystalline form A is obtained by slurring the evaporation residue with methyl acetate or by crystallization from 2-methoxyethanol. The disadvantage of this process is also the necessity of a distillation step, the use of a second solvent for lenalidomide form A crystallization and the use of a high pressure of hydrogen for the hydrogenation step.

Proceeses for preparation of solid forms of lenalidomide are also disclosed in IN47CHE2006 and WO2010/100476 applications.

While several production processes for making lenalidomide crystalline Form A are known in the art, an improvement in this respect is still desirable.

### BRIEF DESCRIPTION OF THE INVENTION

The present invention relates to the discovery of a straightforward process for making crystalline lenalidomide Form A, as defined hereinafter, which is effective in a reliable production environment on an industrial scale without the need of using pressure vessels. The process of the invention is performed in a conventional reaction vessel at atmospheric pressure, without a need of working with hydrogen and hydrogenation catalyst. The desired crystalline lenalidomide precipitates directly from the filtered reaction mixture, without any need of replacing the reaction solvent by a crystallization solvent.

The first object is a process for preparation of crystalline lenalidomide form A of formula (1), 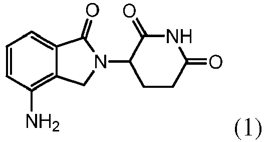 characterized by a XRPD powder diffraction pattern comprising the peaks at about 7.89, 14.35, 14.83, 15.81, 16.23, 20.12, 23.78, 24.09, 25.96 degrees 2 theta, when measured with CuKα1 radiation (λ = 1.54060 Å), comprising:
1. Reduction of 3-(4-nitro-1-oxo-1,3-dihydro-2*H*-isoindol-2-yl)-2,6-piperidinedione of formula (2) with a reducing agent in a mixed solvent comprising a mixture of methanol and N,N-dimethylformamide or N,N-dimethylacetamide, 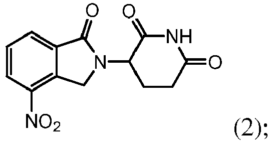 wherein the content of N,N-dimethylformamide or N,N-dimethylacetamide in the mixed solvent is between 20-40% (v/v).
2. Filtering the mixture at a temperature not decreasing below 60°C
3. Adjusting the amount of water in the mixture to 0.4 - 0.6% (v/v)
4. Cooling of the reaction mixture to a temperature lower than 25°C.
5. Isolation of lenalidomide form A.

### BRIEF DESCRIPTION OF THE DRAWINGS

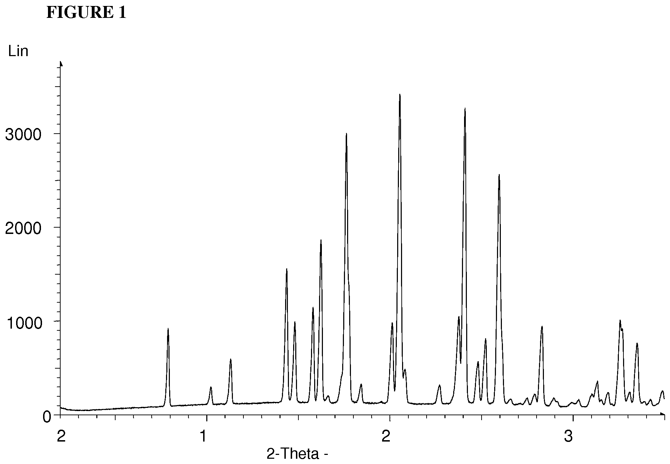

Figure 1 depicts the X-Ray Powder Diffractogram (XRPD) of Form A of Lenalidomide obtainable by the disclosed process according to the Example 2.

### DETAILED DESCRIPTION OF THE INVENTION

The process of the present invention is particularly useful for making crystalline Form A of lenalidomide. Throughout the disclosure and claims, the "Form A" of lenalidomide is a crystalline form characterized by a XRPD powder diffraction pattern comprising , inter alia, the peaks at about 7.89, 14.35, 14.83, 15.81, 16.23, 20.12, 23.78, 24.09, 25.96 degrees 2 theta (±0.2 degrees 2 theta) . Such pattern may be obtained when measured with CuKα1 radiation (λ = 1.54060 Å). The XRPD pattern of lenalidomide Form A obtained by the process of the present invention substantially corresponds to that as disclosed for lenalidomide Form A in WO2005/023192. "Substantially corresponds" is meant to cover variations/differences in the pattern that would not be understood by a worker skilled in the art to represent a difference in crystal structure, but rather differences in technique and sample preparation.

The Form A of lenalidomide produced by the process of the present invention has an excellent batch-to-batch uniformity in the size and shape of the formed crystals. According to one particular aspect of the invention, the lenalidomide Form A crystals produced by the process of the present invention are preferably substantially free from other crystalline forms of lenalidomide. In this respect, the "substantially free" means that less than 10%, and more preferably less than 5% of other crystalline forms are present in the precipitated and/or isolated product comprising the Form A of lenalidomide.

The starting material for the process of the invention is the compound 3-(4-nitro-1-oxo-1,3-dihydro 2*H*-isoindol-2-yl)-2,6-piperidinedione of formula (2). The compound is either commercially available or may be produced according to known procedures, e.g. according to that disclosed in WO98/03502.

In the first step of the process of the present invention, the compound of formula (2) is combined, preferably under stirring, with a reducing agent, a mixed solvent comprising methanol, and N,N-dimethylformamide or N,N-dimethylacetamide, wherein the content of N,N-dimethylformamide of N,N-dimethylacetamide in the mixed solvent is between 20-40%, preferably between 25-33% (v/v), more preferably between 26-30% (v/v) and most preferably between 27-29% (v/v).

The examples of the reducing agent are zinc in combination with acetic acid, zinc in combination with HCl, tin in combination with HCl or iron in combination with HCl. The compound (2) can be also reduced using hydrogen in a presence of a hydrogenation catalyst, for example platinum, nickel, iridium and ruthenium. A combination of zinc and acetic acid are preferably used as a reducing agent.

The concentration of the compound (2) in the mixed solvent is advantageously between 0.09 and 0.30 mmol/ml, preferably between 0.09 and 0.12 mmol/ml.

Metallic zinc and acetic acid, preferably glacial acetic acid, are also combined with the mixed solvent, preferably at a temperature between 5°C and 35°C. The molar ratio between the compound of formula (2) and zinc is between 1:3 and 1:8, preferably between 1:4 and 1:7 and most preferred 1:7. The molar ratio between the compound of formula (2) and acetic acid is between 1:6 and 1:10, preferably between 1:7 and 1:9, most preferred 1:7.5. Zinc is preferably a powderized material with a particle size of less than 150 µm.

The above mixture may preferably also comprise water, typically in an amount of between 0.1-0.5% (v/v), preferably 0.14-0.3 % (v/v) (calculated with respect to the volume of acetic acid). The water may be included in any of reagents in reaction mixture (e.g. in acetic acid) or may be added intentionally to the mixture.

The mixture is heated to a temperature between 5°C and the reflux temperature of the mixture, preferably between 45-68°C and most preferably 60- 65°C and stirred for the time necessary for performing the reduction of starting compound of formula (2) into lenalidomide, preferably for 10-60 minutes, most preferably for about 15 minutes. The conversion of the compound of formula (2) may be monitored by conventional analytical techniques, e.g. by HPLC.

In the second step, the isolation step, the mixture from the step 1 is filtered while being hot, preferably at a temperature not decreasing below 60°C, through suitable filtration equipment. Any conventional filtration technique may be applied. Optionally, the filter cake comprising the unreacted reducing agent and insoluble side products is washed by water or methanol in order to complete the separation of the desired and undesired fractions.

It should be noted that water is a side product of the reaction of the compound (2) with a reducing agent in the step 1. Therefore, the filtrate from the isolation step 2 comprises more water than the starting reaction mixture in the step 1. The amount of water in the filtrate plays certain role. In the presence of higher amounts of water, hydrated forms of lenalidomide may be produced by precipitation thereof from the mixture. In the presence of lower amounts of water and at higher concentrations of N,N-dimethylformamide or N,N-dimethylacetamide, a solvated form of lenalidomide (lenalidomide N,N-dimethylformamide solvate or lenalidomide N,N-dimethylacetamide solvate) may precipitate from the mixture. However, it is nevertheless preferred that the product of the reaction is crystalline Form A, advantageously substantially free from other solid state forms of lenalidomide. Thus, it is advisable to control the content of water, methanol, N,N-dimethylformamide or N,N-dimethylacetamide in the filtrate and to adjust the amounts thereof in respect to the intended purpose.

After the filtration, the filtrate is cooled, optionally under stirring, wherein crystalline lenalidomide Form A precipitates from the solution. The mixture is preferably cooled to a temperature lower than 25°C. More preferably the mixture is seeded by seed crystals of lenalidomide form A while cooling.

The precipitated product can be isolated from the mixture by conventional techniques, e.g. filtering or centrifugation, and can be washed and dried.

If other form than the Form A is produced by the above process, such form can be optionally converted into form A, e.g. by recrystallization under conditions known per se.

For instance, lenalidomide N,N-dimethylformamide solvate or lenalidomide N,N-dimethylacetamide solvate can be converted to the Form A using slurrying lenalidomide N,N-dimethylformamide solvate or lenalidomide N,N-dimethylacetamide solvate in an alcoholic solvent, preferably in n- butanol and stirring of the mixture for the time necessary to transform lenalidomide N,N-dimethylformamide solvate or N,N-dimethylacetamide solvate into Form A.

The lenalidomide Form A prepared by the process of the present invention can be formulated and used in pharmaceutical compositions. For instance, a suitable pharmaceutical composition may comprise lenalidomide Form A and at least one pharmaceutically acceptable excipient.

The lenalidomide Form A prepared by the process of the present invention is suitable for the treatment of a wide range of conditions including autoimmune disease and cancer. In particular, it is useful in the treatment of multiple myeloma, treatment of patients with transfusion-dependent anaemia due to low- or intermediate-1-risk myelodysplastic syndromes associated with an isolated deletion 5q cytogenetic abnormality when other therapeutic options are insufficient or inadequate and treatment of diffuse large B-cell lymphoma. Typically the effective amounts range from 2.5 mg to 7.5 mg, expressed as the amount of lenalidomide base, per day.

The invention will be further described with reference to the following non-limiting examples.

### EXAMPLES

### Example 1

3-(4-nitro-1-oxo-1,3-dihydro-2*H*-isoindol-2-yl)-2,6-piperidinedione (20 g, 69.1 mmol) and zinc dust (18.46 g, 277 mmol) were weighed to three necked 1L flask equipped with magnetic stirrer, condenser and argon inlet. Methanol (460 mL) and N,N-dimethylformamide (160 ml) were added. Resulting gray suspension was mixed with acetic acid (31.1 mL, 553 mmol, water content 0.15% (v/v)) and mixture was heated to 65 °C (bath). Once reaction reached internal reaction temperature 65 °C, the mixture was stirred for 15 minutes.

After 15 minutes, the mixture was filtered hot into a clean three necked flask and resulting bluish clear solution was stirred at 20-25 °C. The suspension was spontaneously cooled to 30 °C over 1 h and then stirred at 20-25 °C for next 10 h.

The suspension was stirred at -5°C for 3 h, when it was filtered off and filter cake was washed with water (3 x 40 ml) and chilled methanol (2x30 ml) giving white powder of lenalidomide. The product was dried for 2 h (45 °C, nitrogen stripping) giving lenalidomide Form A (15 g, 84 % yield, 100% HPLC purity) as a white powder.

### Example 2

3-(4-nitro-1-oxo-1,3-dihydro-2*H*-isoindol-2-yl)-2,6-piperidinedione (1.65 kg, 5.70 mol) and zinc dust (2.67 kg, 40.83 mol) were weighed to methanol (35.10 1) and N,N-dimethylformamide (11.811). Resulting suspension was mixed with acetic acid (2.58 kg, p.a.) and mixture was heated to 60 °C (bath). Once reaction reached internal reaction temperature 60 °C, the mixture was stirred for 15 minutes.

After 15 minutes, the mixture was filtered hot, 0.75 1 of methanol and 0.11 of N,N-dimethylformamide were added and the mixture was stirred at 20-25 °C for 7 hrs.

The suspension was stirred at -5°C for 5 h, when it was filtered off and filter cake was washed with water (2x5 1) and chilled methanol (2x6.311) giving white powder of lenalidomide. The product was dried for 3 h (70 °C, nitrogen stripping) giving lenalidomide Form A (1.1kg, 74 % yield, 99.99%, HPLC purity 99.88%) as a white powder.

An XRPD (Presented in figure 1) that corresponds to Lenalidomide Form A was then obtained using the following measurement conditions:
Bruker-AXS D8 Vario diffractometer with Θ/2Θ geometry (reflection mode), equipped with a Vantec PSD detector

| | |
|---|---|
| Start angle (2θ): | 2.0° |
| End angle (2θ): | 35.0° |
| Scan step width: | 0.02° |
| Scan step time: | between 0.2-2.0 seconds |
| Radiation type: | Cu |
| Radiation wavelengths: | 1.5406Å (Kα1), primary monochromator used |
| Exit slit: | 6.0 mm |
| Focus slit: | 0.2 mm |
| Divergence slit: | Variable (V20) |
| Antiscatter slit: | 11.8 mm |
| Receiving slit: | 20.7 mm |

### Example 3

3-(4-nitro-1-oxo-1,3-dihydro-2*H*-isoindol-2-yl)-2,6-piperidinedione (60 g, 207.44 mmol) and zinc dust (97 g, 1483.63 mmol) were weighed to methanol (1153.5 ml) and N,N-dimethylformamide (459.3 ml). Resulting suspension was mixed with acetic acid (88 g, p.a.) and mixture was heated to 73.8 °C (bath) for 15 minutes.

The mixture was filtered hot and resulting solution was stirred at 20-25 °C for 10 hours.

The suspension was stirred at 0°C for 2 h. The suspension was filtered off and filter cake was washed with water (3 x 240 ml) and chilled methanol (2x272 ml) giving white powder of lenalidomide. The product was dried for 3 h (65 °C, nitrogen stripping) giving lenalidomide Form A (30.94 g, 57.5 % yield, HPLC purity 99.13%) as a white powder.

### Example 4

Lenalidomide N,N-dimethylformamide solvate (0.305 g, 1.176 mmol) was weighed into 10 ml reaction vial equipped with magnetic stirring bar. N-butanol (5 ml) was added. The vial was immersed in to an oil bath and heated to 85 °C (bath) with stirring for 3 h. Mixture was then cooled to 20-25 °C and solid material was isolated by suction and washed with n-butanol (5 ml). Lenalidomide was obtained in form A (99% yield, the purity of obtained lenalidomide form A corresponds to the purity of starting lenalidomide N,N-dimethylformamide solvate).

### Example 5

Lenalidomide N,N-dimethylformamide solvate (0.305 g, 1.176 mmol) was weighed into 10 ml reaction vial equipped with magnetic stirring bar. 2-Propanol (5 ml) was added. The vial was immersed in to an oil bath and heated to 85 °C (bath) with stirring for 3 h. Mixtures were then cooled to 20-25 °C and solid material was isolated by suction and washed with 2-Propanol (5 ml). Lenalidomide was obtained in form A (99% yield, the purity of obtained lenalidomide form A corresponds to the purity of starting lenalidomide N,N-dimethylformamide solvate).

### Example 6

3-(4-nitro-1-oxo-1,3-dihydro-2*H*-isoindol-2-yl)-2,6-piperidinedione (3 g, 10.4 mmol) and zinc dust (2.79 g, 42.67 mmol) were weighed to three necked 1L flask equipped with magnetic stirrer, condenser and argon inlet. Methanol (39 mL) and N,N-dimethylacetamide (14 ml) were added. Resulting gray suspension was mixed with acetic acid (5.04 g, 84 mmol, water content 0.15% (v/v)) and mixture was heated to 65 °C (bath). Once reaction reached internal reaction temperature 65 °C, the mixture was stirred for 30 minutes.

After 30 minutes, the mixture was filtered hot into a clean three necked flask and resulting bluish clear solution was stirred at 20-25 °C. The suspension was spontaneously cooled to 30 °C over 1 h and then stirred at 20-25 °C for next 10 h.

The suspension was stirred at -5°C for 3 h, when it was filtered off and filter cake was washed with water (3 x 40 ml) and chilled methanol (2x30 ml) giving white powder of lenalidomide. The product was dried for 2 h (45 °C, nitrogen stripping) giving lenalidomide Form A (1.34 g, 50 % yield, 98% HPLC purity) as a white powder.

### Example 7

Lenalidomide N,N-dimethylacetamide solvate (0.5 g, 1.444 mmol) was weighed into 10 ml reaction vial equipped with magnetic stirring bar. N-butanol (5 ml) was added. The vial was immersed in to an oil bath and heated to 85 °C (bath) with stirring for 3 h. Mixture was then cooled to 20-25 °C and solid material was isolated by suction and washed with n-butanol (5 ml). Lenalidomide was obtained in form A (99% yield, the purity of obtained lenalidomide form A corresponds to the purity of starting lenalidomide N,N-dimethylacetamide solvate).

## Claims

1. A process for making crystalline lenalidomide form A of formula (1) 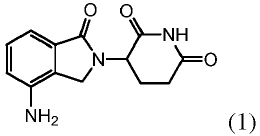 **characterized by** a XRPD powder diffraction pattern comprising the peaks at about 7.89, 14.35, 14.83, 15.81, 16.23, 20.12, 23.78, 24.09, 25.96 degrees 2 theta, when measured with CuKα1 radiation (λ = 1.54060 Å), the process comprising:
a. Reduction of 3-(4-nitro-1-oxo-1,3-dihydro-2*H*-isoindol-2-yl)-2,6-piperidinedione of formula (2) with a reducing agent in a mixed solvent comprising a mixture of methanol and N,N-dimethylformamide or N,N-dimethylacetamide, 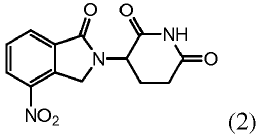 wherein the content of N,N-dimethylformamide or N,N-dimethylacetamide in the mixed solvent is between 20-40% (v/v).
b. Filtering the mixture at a temperature not decreasing below 60°C
c. Adjusting the amount of water in the mixture to 0.4 - 0.6% (v/v)
d. Cooling of the reaction mixture to a temperature lower than 25°C.
e. Isolation of lenalidomide form A

2. The process according to claim 1, wherein the content of N,N-dimethylformamide or N,N-dimethylacetamide in the mixed solvent is between 25-33% (v/v).

3. The process according to claim 1 and 2 wherein the reducing agent is zinc and acetic acid.

4. The process according to claim 3, wherein the molar ratio between the compound of formula (2) and acetic acid is between 1:6 and 1:10.

5. The process according to claim 3, wherein the molar ratio between the compound of formula (2) and acetic acid is 1:7.5.

6. The process according to any of claims 3 to 5, wherein the molar ratio between the compound of formula (2) and zinc is between 1:3 and 1:8.

7. The process according to any of claims 3 to 5, wherein the molar ratio between the compound of formula (2) and zinc is 1:7.

8. The process according to any of claims 1 to 7, wherein the concentration of the compound (2) in the mixed solvent is between 0.09 and 0.30 mmol/ml.

9. The process according to any of claims 1 to 7, wherein the concentration of the compound (2) in the mixed solvent is between 0.09 and 0.12 mmol/ml.

10. The process according to any of claims 1 to 9, wherein the reaction mixture in the reduction step comprises water in an amount between 0.1 and 0.5% (v/v) in respect to the volume of acetic acid.

## Patentansprüche

1. Verfahren zur Herstellung einer kristallinen Form von Lenalidomid A der Formel (1) 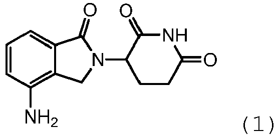 **gekennzeichnet durch** ein XRPD-Pulverbeugungsmuster, das Peaks bei ungefähr 7.89, 14.35, 14.83, 15.81, 16.23, 20.12, 23.78, 24.09, 25.96 Grad 2-Theta enthält, wenn eine Messung mit CuKα1-Strahlung (λ = 1,54060 Å) erfolgt, das Verfahren umfassend:
a. Reduktion von 3-(4-Nitro-1-Oxo-1,3-Dihydro-2*H*-Isoindol-2-yl)-2,6-Piperidinedion der Formel (2) mit einem Reduktionsmittel in einem Lösungsmittelgemisch, das ein Gemisch aus Methanol und N,N-Dimethylformamid oder N,N-Dimethylacetamid enthält, 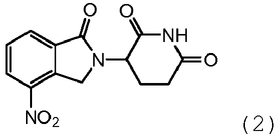 wobei der Gehalt von N,N-Dimethylformamid oder N,N-Dimethylacetamid in dem Lösungsmittelgemisch zwischen 20 - 40 % (v/v) beträgt.
b. Filtern des Gemisches bei einer Temperatur, die 60 °C nicht unterschreitet
c. Anpassen der Wassermenge in dem Gemisch auf 0,4 - 0.6 % (v/v)
d. Abkühlen des Reaktionsgemisches auf eine Temperatur, die niedriger als 25 °C ist.
e. Isolierung der Form von Lenalidomid A

2. Verfahren nach Anspruch 1, wobei der Gehalt von N,N-Dimethylformamid oder N,N-Dimethylacetamid in dem Lösungsmittelgemisch zwischen 25 - 33 % (v/v) beträgt.

3. Verfahren nach Anspruch 1 und 2, wobei das Reduktionsmittel Zink und Essigsäure ist.

4. Verfahren nach Anspruch 3, wobei das Molarverhältnis zwischen der Verbindung der Formel (2) und Essigsäure zwischen 1:6 und 1:10 beträgt.

5. Verfahren nach Anspruch 3, wobei das Molarverhältnis zwischen der Verbindung der Formel (2) und Essigsäure 1:7.5 beträgt.

6. Verfahren nach einem der Ansprüche 3 bis 5, wobei das Molarverhältnis zwischen der Verbindung der Formel (2) und Zink zwischen 1:3 und 1:8 beträgt.

7. Verfahren nach einem der Ansprüche 3 bis 5, wobei das Molarverhältnis zwischen der Verbindung der Formel (2) und Zink 1:7 beträgt.

8. Verfahren nach einem der Ansprüche 1 bis 7, wobei die Konzentration der Verbindung (2) in dem Lösungsmittelgemisch zwischen 0.09 und 0.30 mmol/ml beträgt.

9. Verfahren nach einem der Ansprüche 1 bis 7, wobei die Konzentration der Verbindung (2) in dem Lösungsmittelgemisch zwischen 0.09 und 0.12 mmol/ml beträgt.

10. Verfahren nach einem der Ansprüche 1 bis 9, wobei das Reaktionsgemisch in dem Reduktionsschritt Wasser in einer Menge zwischen 0.1 und 0.5 % (v/v) in Bezug auf das Volumen Essigsäure enthält.

## Revendications

1. Procédé de fabrication d'une forme cristalline A de lénalidomide selon la formule (1) 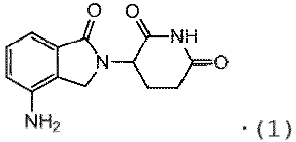 **caractérisée par** un diagramme de diffraction X sur poudre XRPD comprenant les pics à environ 7.89, 14.35, 14.83, 15.81, 16.23, 20.12, 23.78, 24.09, 25.96 degrés 2 thêta, lors de la mesure avec un rayonnement CuKα1 radiation (λ = 1,54060 Å), le procédé comprenant :
a. une réduction de la 3-(4-nitro-1-oxo-1,3-dihydro-2*H-*isoindol-2-yl)-2,6-pipéridinedione selon la formule (2) avec un agent de réduction dans un solvant mixte comprenant un mélange de méthanol et de N, N-diméthylformamide ou de N, N-diméthylacétamide, 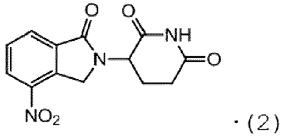 où la teneur en N, N-diméthylformamide ou en N, N-diméthylacétamide dans le solvant mixte étant entre 20 - 40 % (v/v).
b. une filtration du mélange à une température n'étant pas inférieure à 60 °C
c. l'ajustement de la quantité d'eau dans le mélange à 0.4 - 0.6 % (v/v)
d. le refroidissement du mélange réactionnel à une température inférieur à 25 °C.
e. l'isolation du lénalidomide de forme A

2. Procédé selon la revendication 1, dans lequel la teneur en N, N-diméthylformamide ou en N, N-diméthylacétamide dans le solvant mixte est entre 25 - 33 % (v/v).

3. Procédé selon les revendications 1 et 2, dans lequel l'agent de réduction est du zinc et de l'acide acétique.

4. Procédé selon la revendication 3, dans lequel le ratio molaire entre le composé selon la formule (2) et l'acide acétique est entre 1 : 6 et 1 : 10.

5. Procédé selon la revendication 3, dans lequel le ratio molaire entre le composé selon la formule (2) et l'acide acétique est de 1 : 7.5.

6. Procédé selon l'une quelconque des revendications 3 à 5, dans lequel le ratio molaire entre le composé selon la formule (2) et le zinc est entre 1 : 3 et 1 : 8.

7. Procédé selon l'une quelconque des revendications 3 à 5, dans lequel le ratio molaire entre le composé selon la formule (2) et le zinc est de 1 : 7.

8. Procédé selon l'une quelconque des revendications 1 à 7, dans lequel la concentration du composé (2) dans le solvant mixte est entre 0.09 et 0.30 mmol/ml.

9. Procédé selon l'une quelconque des revendications 1 à 7, dans lequel la concentration du composé (2) dans le solvant mixte est entre 0.09 et 0.12 mmol/ml.

10. Procédé selon l'une quelconque des revendications 1 à 9, dans lequel le mélange réactionnel dans l'étape de réduction comprend de l'eau en une quantité entre 0.1 et 0.5 % (v/v) par rapport au volume d'acide acétique.
